# EUROPÄISCHE PATENTANMELDUNG

(11) **EP 0 976 758 A1**
(43) Veröffentlichungstag der Anmeldung: **02.02.2000**
(21) Anmeldenummer: 99113358.8
(22) Anmeldetag: 09.07.1999
(51) Int. Cl.: C07K 1/26, G01N 27/447

(54) **Verwendung von porösen, neutral oder negativ geladenen Membranmaterialien als Beladungsmaterialien bei der Gelelektrophorese**

(30) Priorität: 10.07.1998 DE 19830989
(71) Anmelder: LION Bioscience AG, 69120 Heidelberg (DE)
(72) Erfinder: Erfle, Holger, 69207 Sandhausen (DE); Remmel, Bettina, 69221 Dosssenheim (DE); Schütte, Dagmar, 69221 Dossenheim (DE)
(74) Vertreter: Schüssler, Andrea, Dr.

(57) **Zusammenfassung**

Die vorliegende Erfindung betrifft die Verwendung von porösen hydrophilen Membranmaterialien, die an ihrer Oberfläche neutral oder negativ geladen sind, als Beladungsmaterialien bei der Gelelektrophorese von Nukleinsäuren oder Proteinen, die mit einem hydrophoben Farbstoff markiert sind.

## Beschreibung

Die vorliegende Erfindung betrifft die Verwendung von porösen hydrophilen Membranmaterialien, die an ihrer Oberfläche neutral oder negativ geladen sind, als Beladungsmaterialien bei der Gelelektrophorese von Nukleinsäuren oder Proteinen, die mit einem hydrophoben Farbstoff markiert sind.

Bei den bisher bekannten Verfahren zum Beladen einer Gelelektrophorese-Apparatur, wird das aufzutragende Probenmaterial in Taschen eingebracht, die in dem verwendeten Gel ausgebildet worden sind. Geltaschen werden dabei auf zwei Arten erzeugt: (1) Plastikkämme, die Zähne in Form der auszubildenden Geltaschen besitzen, werden als Platzhalter ins unpolymerisierte Gel eingeführt und nach der Polymerisation des Gels herausgezogen und (2) "Haifischzahnkämme", d.h. Plastikkämme, die die Form der Geltaschenwände besitzen, werden auf das bereits auspolymerisierte Gel aufgesetzt. Gerade die zweite Methode eignet sich aber nur für sehr dünne Gele. Das Beladen der ausgeformten Geltaschen kann per Hand geschehen, d.h. das Probenmaterial wird mit einer Einzel- oder Multipipette aufgenommen und in die Geltaschen eingebracht. Inzwischen gibt es jedoch auch Pipettier-Roboter, die mit einer größeren Genauigkeit den Probenmaterial-Auftrag vornehmen. Allgemein bestehen aber bei den bisher bekannten Methoden der Gelelektrophorese folgende Probleme:
- aufwendiges Spülen der ausgebildeten Taschen,
- Beschädigung des Trennmittels (Gels), insbesondere wenn ein "Haifischzahnkamm" verwendet wird,
- Diffusion der Proben aus den Taschen und dadurch Einsetzen eines höchst unwillkommenen Vermischvorgangs,
- Beladen schmaler Trennmitteltaschen sehr schwierig,
- schwieriges Einführen des Haifischzahnkamms,
- Beschädigung der Trennmitteltaschen, wodurch weniger Beladungsstellen als ursprünglich beabsichtigt zur Verfügung stehen,
- wenige Beladungsstellen (ca. 20-80), die pro Probenbearbeitung zur Verfügung stehen.

Es gibt auch bereits Modifikationen der obigen Gelelektrophoreseverfahren. Bei diesen wird eine Geltasche geformt, in die ein mit der aufzutrennenden Probe getränkter Zahnkamm eingebracht wird (US-A-5,405,516; WO 96/27787). Durch Anlegen von Spannung kommt es zur Abgabe der Probe in das Gel und zur gewünschten Auftrennung der Probe. Als mögliche Materialien für den Zahnkamm wurden poröse Materialien, z.B. Cellulose-Mischester und Nylon, genannt (Erfle et al., Nucleic Acids Research, 1997, Vol. 25, No. 11, S. 2229-2230).
Der Einsatz dieser modifizierten Gelelektrophorese-Methode für die DNA-Sequenzierung nach Sanger, wo unterschiedliche Sequenzreaktionen unterschiedlich mit hydrophoben Farbstoffen markiert sind, brachte jedoch unzufriedenstellende Ergebnisse, die teilweise nicht auswertbar waren.

Die Aufgabe der vorliegenden Erfindung besteht deshalb darin, eine Möglichkeit bereitzustellen, mit die Protein- oder Nukleinsäure-Auftrennung verbessert werden kann, wenn diese mit hydrophoben Farbstoffen markiert worden sind.

Diese Aufgabe wird durch die Gegenstände der Patentansprüche gelöst.

Von den Erfindern wurde experimentell ermittelt, daß poröse Materialien (Membranen), welche an ihrer Oberfläche hydrophil und ungeladen oder negativ geladen waren, bei der Auftrennung von Nukleinsäuren/Proteinen, die mit hydrophoben Farbstoffen markiert waren, besonders gute Ergebnisse lieferten.

Unter einem porösen Material soll erfindungsgemäße ein Material verstanden werden, dessen Porengröße derart ist, daß eine darauf aufgetragene Probe zumindest bei der Probenzugabe darauf und bei der Probenabgabe an ein Trennmittel, das sich z.B. in einer Elektrophorese-Apparatur befindet, im porösen Material in flüssiger Phase aufgrund von Kapillarkräften daran gehalten wird. Das poröse Material liegt vorzugsweise in Form eines Probenzuführteils, z.B. in Form eines Kamms, der Zähne aufweisen kann, aber nicht muß, vor. Dieses Probenzuführteil kann ganz oder teilweise aus dem porösen Material bestehen und ist an die Form einer Elektrophoreseapparatur angepaßt.

Zwischenzeitlich kann das Probenmaterial in getrockneter Form in den Poren des porösen Materials vorliegen. Reaktionen zur chemischen Bindung des Probenmaterials an das Probenzuführteil sind ebensowenig erforderlich wie chemische Reaktionen zum Ablösen des Probenmaterials von dem Probenzuführteil. Da die Kapillarkräfte das Probenmaterial in der flüssigen Phase bei der Probenabgabe bis zum Übertritt in die Elektrophorese-Apparatur halten, besteht auch keine Einschränkung in Bezug auf die Orientierung der Elektrophorese-Apparatur. Der Abschnitt des Probenzuführteils, das das Probenmaterial aufnimmt, besteht vorzugsweise durchgängig aus pörosem Material. Die übrigen Abschnitte des Probenzuführteils unterliegen keiner Beschränkung und können aus den für diese Gegenstände üblichen Plastikmaterialien sein. Andererseits kann das Probenzuführteil auch vollständig aus dem porösem Material bestehen.

Für die Erfindung verwendbare poröse Membranen können ausgewählt werden aus:
- - Nylon: (ungeladenes hydrophiles Grundmaterial, das ggf. durch negativ geladene Gruppen, z.B. Carboxyl- oder Hydroxylgruppen, oberflächenmodifiziert ist)
- - Polyethersulfone: (ungeladenes hydrophiles Grundmaterial, das ggf. durch negativ geladene Gruppen, z.B. Carboxyl- oder Hydroxylgruppen oberflächenmodifiziert ist)
- - hydrophil modifiziertes Polyvinylidenfluorid: (hydrophobes Grundmaterial, das durch Oberflächenmodifikation einen hydrophilen Charakter erhält)
- - Cellulose-Acetat: (ungeladenes hydrophiles Grundmaterial, das ggf. durch negativ geladene Gruppen, z.B. Carboxyl- oder Hydroxylgruppen oberflächenmodifiziert ist)
- - Cellulose-Mischester: (ungeladenes hydrophiles Grundmaterial, das ggf. durch negativ geladene Gruppen, z.B. Carboxyl- oder Hydroxygruppen oberflächenmodifiziert ist)
- - regenerierte Cellulose: (ungeladenes hydrophiles Grundmaterial, das ggf. durch negativ geladene Gruppen, z.B. Carboxyl- oder Hydroxylgruppen oberflächenmodifiziert ist)

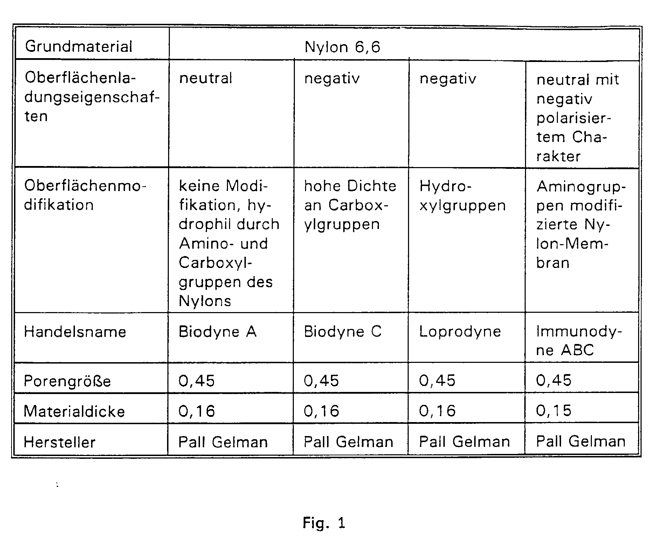

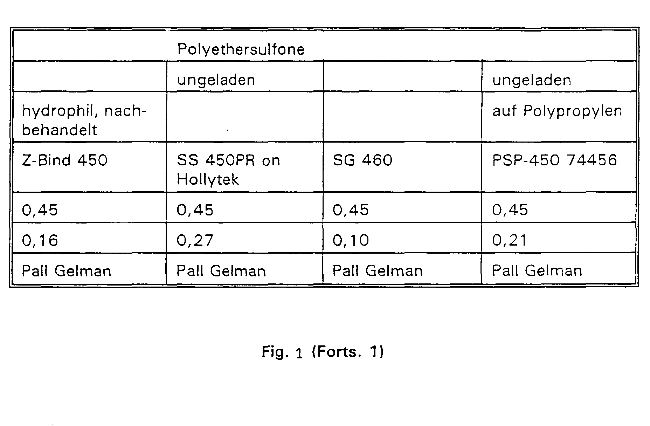

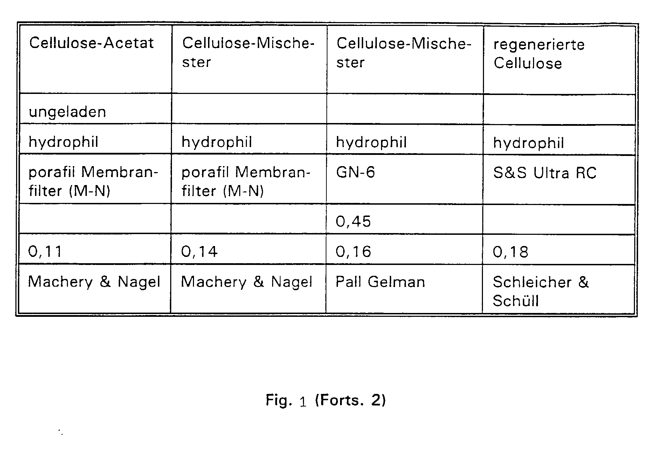

Verfahren zur Oberflächenmodifikation sowie die Auswahl geeigneter modifizierender Gruppen für diese Membranen sind dem Fachmann hinreichend bekannt. Außerdem sind die meisten Membranen in geeignet modifizierter Form bereits käuflich erhältlich (vgl. Fig. 1).

Bevorzugt wird ein poröses Material mit einer mittleren Porengröße unter 100 µm, vorzugsweise unter 10 µm, am besten unter 1-2 µm, eingesetzt bzw. mit einer mittleren Porengröße zwischen 1,2 und 0,2 µm, am besten bei etwa 0,45-0,50 µm. Derartiges poröses Material weist für die üblicherweise eingesetzten Probenmaterialien, insbesondere im biochemischen Bereich, ausreichend hohe Kapillarkräfte auf.

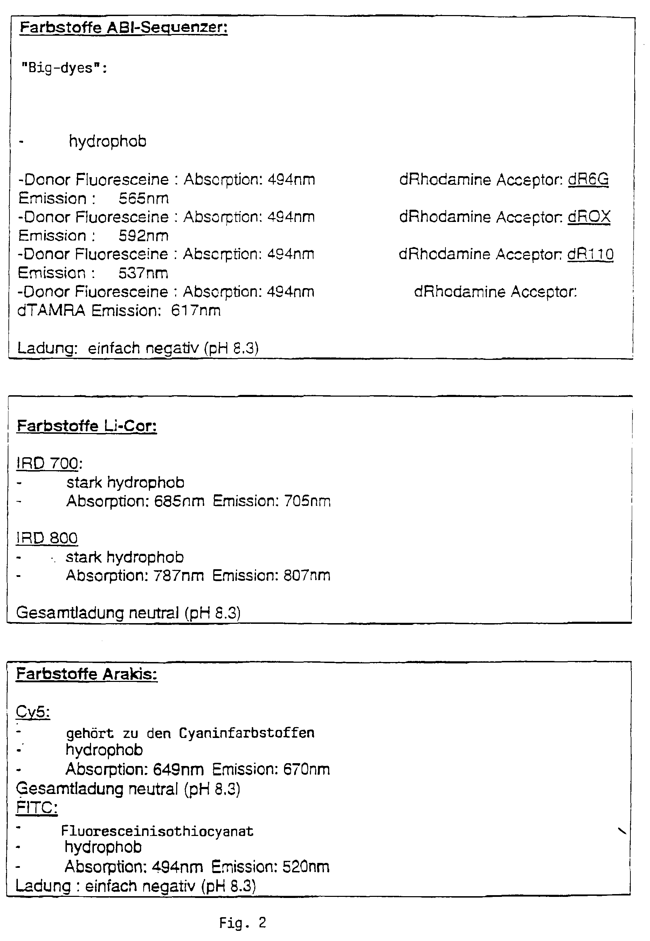

Unter dem Begriff "hydrophobe Farbstoffe" sollen alle farbgebenden Substanzen verstanden werden, die hydrophobe Eigenschaften haben und für die Markierung von Biomolekülen eingesetzt werden können. Je nach gewählter Auftrennungs- bzw. Sequenzierungsmethode müssen verschiedene Farbstoffe ausgewählt werden. Eine Zusammenstellung bevorzugter Farbstoffe sind in Fig. 2 gezeigt. Von den Erfindern wurden eine Reihe von Membranen als poröse Materialien für die Elektrophorese getestet. Es stellte sich heraus, daß eine Nylonmembran, welche an ihrer Oberfläche mit zusätzlichen Hydroxyl- oder Carboxylgruppen modifiziert worden ist, bei Nukleinsäuresequenzierungen, bei dem die stark hydrophoben Farbstoffe IRD700 oder IRD800 (Fa. Li-Cor) eingesetzt wurden, sehr gute Ergebnisse liefert. Dies ist beispielsweise "Biodyne C" oder "Loprodyne" der Fa. Pall gelman. Wurde die Markierung mit den Farbstoffen FITC (Fa. Pharmacia-Upjohn) oder Cy5 (Fa. Pharmacia-Upjohn) vorgenommen, stellte sich eine hydrophile Grundmatrix ohne zusätzliche Ladungsträger (z.B. eine nicht oberflächenmodifizierte Nylonmembran oder ein Cellulose-Mischester) als am bevorzugtesten heraus. Dies ist beispielsweise "Biodyne A" (Fa. Pall gelman) oder "Porafil Membranfilter M-N" (Fa. Machery-Nagel).

Der positive Effekt der erfindungsgemäß verwendeten Membranen, insbesondere bei Verwendung von "Biodyne A" oder "Biodyne C" oder "Loprodyne", kann weiter gesteigert werden, wenn bei der DNA-Sequenzierung Sequenzierungspuffer verwendet werden, die Ammoniumsulfat enthalten und die für die Sequenzierung notwendigen A/T/G/C-Terminationsmixe dITP enthalten. Besonders gute Ergebnisse werden mit folgenden Puffern und Nukleotidmixen erreicht:

### 10 x Sequenzierungspuffer:

30-70 (bevorzugt 50) mM MgCl₂
100-150 (bevorzugt 125) mM Tris-HCl
100-200 (bevorzugt 150) mM (NH₄)₂SO₄

### A-Mix:

0,5-2 (bevorzugt 1) mM dATP
0,5-3 (bevorzugt 1) mM dCTP
0,1-0,5 (bevorzugt 0,4) mM dGTP
1-2 (bevorzugt 1,6) mM dITP
0,5-2 (bevorzugt 1) mM dTTP
1-10 (bevorzugt 5) µM ddATP

### C-Mix:

0,5-2 (bevorzugt 1) mM dATP
0,5-3 (bevorzugt 1) mM dCTP
0,1-0,5 (bevorzugt 0,4) mM dGTP
1-2 (bevorzugt 1,6) mM dITP
0,5-2 (bevorzugt 1) mM dTTP
1-10 (bevorzugt 5) µM ddCTP

### G-Mix:

0,5-2 (bevorzugt 1) mM dATP
0,5-3 (bevorzugt 1) mM dCTP
0,1-0,5 (bevorzugt 0,4) mM dGTP
1-2 (bevorzugt 1,6) mM dITP
0,5-2 (bevorzugt 1) mM dTTP
1-10 (bevorzugt 5) µM ddGTP

### T-Mix:

0,5-2 (bevorzugt 1) mM dATP
0,5-3 (bevorzugt 1) mM dCTP
0,1-0,5 (bevorzugt 0,4) mM dGTP
1-2 (bevorzugt 1,6) mM dITP
0,5-2 (bevorzugt 1) mM dTTP
1-10 (bevorzugt 5) µM ddTTP

Für die in der Anmeldung bevorzugteste Anwendung, die DNA-Sequenzierung, seien als Analysemethoden beispielhaft die DNA-Detektion mittels eines Sequenzierautomaten der Fa. Li-Cor oder Fa. ABI oder Arakis-EMBL genannt. Der Sequenzierautomat der Fa. Fa. Li-Cor besteht aus einer vertikalen Gelelektrophorese-Apparatur, wobei am Ende ein Laser zum Anregen der mit einem Farbstoff markierten Nukleinsäure und ein Detektor (Photomultiplier, Photodiode) zur Aufnahme des emittierten Lichts vorhanden ist. analysiert. Das Arakis-EMBL-System benutzt Detektorarrays, d.h. das Aufnahmesystem ist stationär und besteht aus 384 in Reihe angeordneten Einzeldioden auf 300 mm.

Bei der Gelchromatografie geht es bevorzugt um die Auftrennung von Biomolekülen (z.B. Nukleinsäuren und Proteine), z.B. bei Southern, Northern bzw. Western Blot sowie bei Sequenzierungsrektionen.

Ein weiterer Aspekt der Erfindung ist auf ein Verfahren zur Zuführung von Probenmaterial (z.B. Nukleinsäuren oder Proteinen) gerichtet, das mit einem hydrophoben Farbstoff markiert ist, mittels eines Probenzuführteils (unabhängig davon, ob dieses Zähne aufweist oder nicht) aus hydrophilem Membranmaterial, das an der Oberfläche neutral oder negativ geladen, wobei das Probenmaterial mit einem definierten Volumen zwischen 10 nl und 5 µl aktiv manuell oder automatisiert beladen wird. Dabei kann die aktive, manuelle oder automatisierte Beladung mittels einer Nadel, mittels einer Piezopipette, mittels einer Kapillare, mittels eines Dispensers oder mittels einer Pipettenspitze geschehen.

Um eine Probenabgabe vom Probezuführteil an die Probenbearbeitungsvorrichtung zu erzwingen, kann man ein den porösen Materialabschnitt durchsetzendes elektrisches Feld erzeugen, um einen Fluß elektrisch geladener Moleküle, Makromoleküle oder Teilchen des Probenmaterials vom porösen Materialabschnitt in die Probenbearbeitungsvorrichtung zu bewirken. Die Stärke des elektrischen Felds wird in Abhängigkeit von der elektrischen Ladung so gewählt, daß die Kapillarkräfte überwunden wurden. Besonders vorteilhaft ist die Anwendung dieses Verfahrensschritts bei der Elektrophorese, da dort sowieso die Mittel zur Erzeugung des elektrischen Feldes vorgesehen sind.

Ist das Probenzuführteil vollständig aus porösem Material, ist es möglich, das Probenmaterial an beliebiger Stelle auf dem Probenzuführteil aufzutragen und die sich ausgebildeten Kapillarkräfte für den Probentransport sorgen zu lassen. Es versteht sich aber von selbst, daß Proben nur auf porösen Materialabschnitten aufgetragen werden und das Probenzuführteil nachfolgend bis zur Kante aus porösem Material besteht, um den Übertritt der Probe ins Trennmittel zu gewährleisten.

Auf dem Probenzuführteil kann man aber auch die Möglichkeit des Mischens gezielt ausnutzen, indem man das poröse Material an verschiedenen Stellen oder nacheinander an der gleichen Stelle mit den zu vermischenden Substanzen benetzt.

Es können, wie erwähnt, jeweils für sich gesonderte Materialabschnitte eingesetzt werden mit dem Vorzug strikter Trennung, ohne die Gefahr eines Übersprechens. Herstellung und Handhabung der Materialabschnitte vereinfacht sich jedoch dann wesentlich, wenn das Probenzuführteil erfindungsgemäß einen Materialabschnittsträger umfaßt, welcher die Materialabschnitte in einer der geometrischen Anordnung der Probenannahmestellen entsprechenden Anordnung trägt. Es ist auch möglich auf dem porösen Material des Probenzuführteils Abschnitte zu definieren, auf die die Probe manuell oder automatisiert aktiv aufgetragen werden kann und welche, die für einen Probenmaterialauftrag, z.B. durch Aufbringen einer Beschichtung, gesperrt sind. Die gesperrten Bereiche sind dabei vorzugsweise hydrophob, während die Probenauftragsbereiche vorzugsweise hydrophil sind. Diese unterschiedlichen Bereiche können mehr oder weniger groß sein und können abwechseln.

Die Probenannahme des Probenmaterials durch die Probenbearbeitungseinrichtung unter Einwirkung des genannten elektrischen Feldes setzt eine elektrische Ladung der zu bewegenden Substanzen voraus. Diese Ladung kann bei biologischen Makromolekülen in gewünschter Weise erhalten werden, indem man den pH-Wert der flüssigen Phase in entsprechender Weise einstellt.

Es hat sich herausgestellt, daß unter dem elektrischen Feld praktisch das gesamte Probenmaterial vom Probenzuführteil abgegeben und der Probenbearbeitungseinrichtung zugeführt wird. Dies eröffnet die Möglichkeit, daß man das Probenzuführteil mehrmals hintereinander zur Zuführung von Probenflüssigkeit einsetzt. Der Aufwand für die Durchführung des erfindungsgemäßen Verfahrens wird hierdurch wiederum reduziert.

Das Probenzuführteil kann auf eine Vielzahl von Arten beladen werden, von denen beispielhaft die folgenden Auftragsvorrichtungen erwähnt seien:
- - Piezopipette:: Volumina bis in den Nanoliterbereich können aufgebracht werden,
- - Kapillare:: Schmalste Röhrchen nehmen durch Kapillarkräfte Probenmaterial auf, das durch Berührung mit dem porösen Material auf dem Probenzuführteil an dieses abgegeben wird,
- - Nadel:: Die Nadel nimmt das Probenmaterial durch Adhäsionskräfte an der Spitze auf und gibt es bei Berührung mit dem porösen Material des Probenzuführteils an dieses wieder ab,
- - Dispenser:: Arbeitet nach dem Unterdruck-Prinzip, Volumina von 0,5 bis 1 µl können genau aufgenommen und abgegeben werden.

Bevorzugt erfolgt der Auftrag des Probenmaterials auf das Probenzuführteil automatisiert, da damit erwartungsgemäß eine genauere Auftragsfront erreicht werden kann, was gerade für Sequenzierungsreaktionen wünschenswert ist. Hierzu kann ein Pipettierroboter verwendet werden. Gängige Geräte sind der Biomek 2000 (Beckman Instruments) oder der Tekan Genesis RSP 200 (Fa. Tekan). Aber auch die manuelle Aufbringung des Probenmaterials ist möglich. Bevorzugt von den obigen Auftragungsmöglichkeiten ist der Auftrag mittels Piezopipette. d.h. einem Dispenser ähnlich zu denen, die als Tintenstrahldrucker bekannt sind.

Die Erfindung wird weiter anhand der Figuren beschrieben, welche zeigen:
- Fig. 1:: Membran-Grundmaterialien
- Fig. 2:: Hydrophobe Farbstoffe

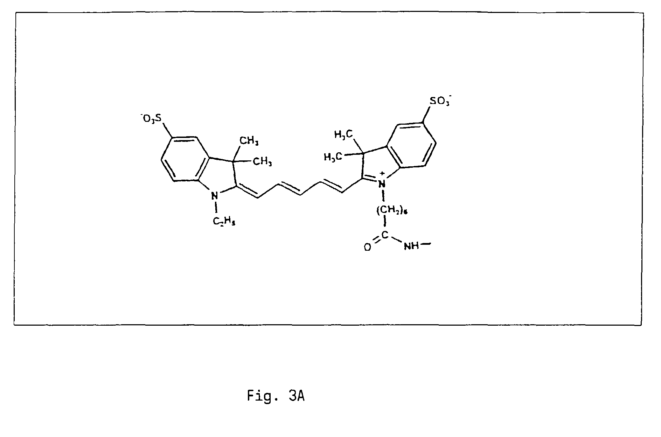

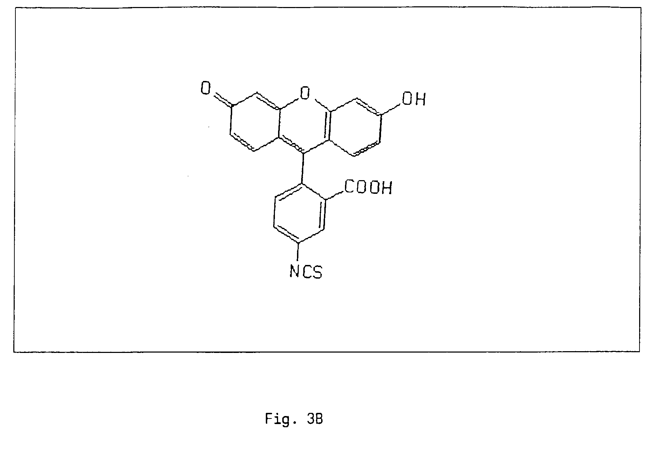

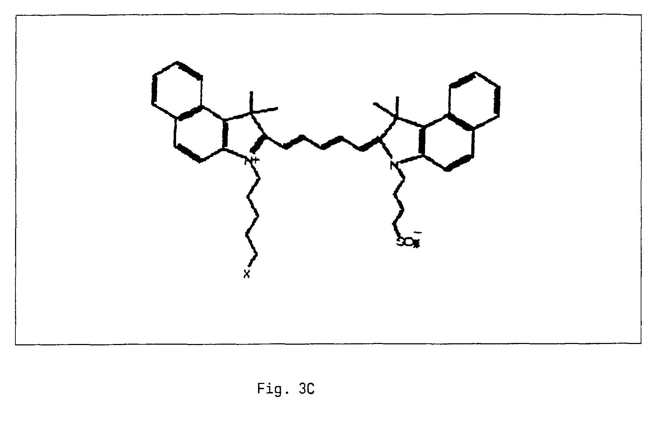

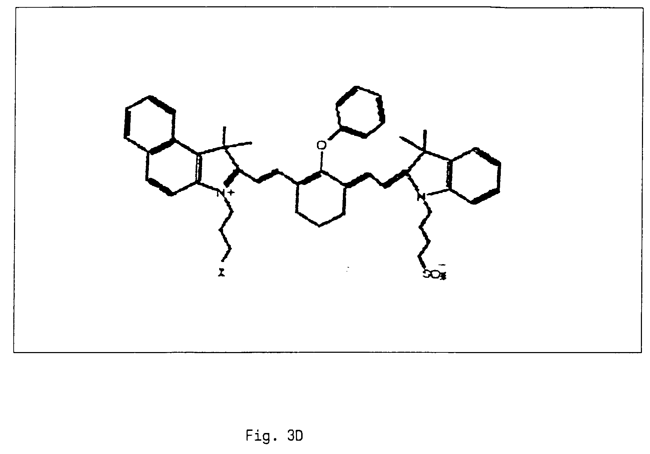

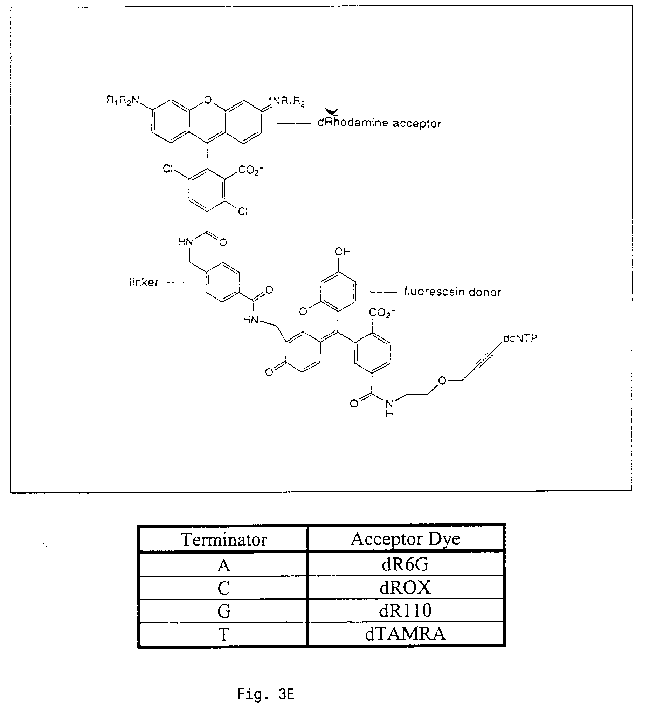

- Fig. 3:: A: Farbstoff Cy5
B: Farbstoff FITC
C: Farbstoff IRD 700
D: Farbstoff IRD 800
E: Farbstoff "ABI Big Dye"-Terminator
- Fig. 4:: Sequenzierungsergebnis
Eine mit IRD 700 markierte DNA-Sequenzreaktion wurde auf eine modifizierte Nylon-Membran geladen, welche zusätzliche Carboxylgruppen trägt (Biodyne C^{R}) trägt. Das Gelbild zeigt eine gute Auflösung der Reaktion, sowie starke Signale.
- Fig. 5:: Sequenzierungsergebnis
Eine mit Cy5 markierte DNA-Sequenzreaktion wurde auf eine modifizierte Nylonmembran geladen, welche zusätzliche Hydroxylgruppen trägt (Loprodyne^{R}) trägt. Das Gelbild zeigt eine gute Auflösung der Reaktion sowie starke Signale
- Fig. 6:: Sequenzierungsergebnis
Eine mit IRD 700 markierte DNA-Sequenzreaktion wurde auf eine modifizierte Nylonmembran geladen, welche eine hohe Dichte an quaternären Ammoniumgruppen (= positive Oberflächenladung; Biodyne B^{R}) trägt. Das Gelbild zeigt fast keine Signale.
- Fig. 7:: Sequenzierungsergebnis
Eine mit Cy5 markierte DNA-Sequenzreaktion wurde auf eine modifizierte Nylonmembran geladen, welche eine hohe Dichte an quaternären Ammoniumgruppen (= positive Oberflächenladung; Biodyne B^{R}) trägt. Das Gelbild zeigt fast keine Signale.

Die Erfindung wird weiter anhand des Beispiels beschrieben:

### Beispiel:

### Sequenzierung für Li-Cor

### Li-Cor-Reaktionen:

- 1 µl AmpliTaq FS DNA Polymerase:
- 1 µl DNA: (pBluescript-Derivat; Insert-Größe ca. 2 kB) (1 µg/µl)
- 1 µl Reverse Primer: (2 µM) mit IRD-700 markiert (5'-CAGT GAA ACA GCT ATG AC-3')
- 1 µl Universal Primer: (2 µM) mit IRD-800 markiert (5'-CGA CGT TGT AAA ACG ACG GCC AGT-3')
- 2 µl 10 x Sequenzierungspuffer: (50 mM MgCl₂/125 mM Tris/150 mM (NH₄)₂SO₄)
- 14 µl H₂O:

Es wurden jeweils 4,5 µl des Gemischs auf vier Röhrchen verteilt, welche mit A, C, G oder T beschriftet waren und 2 µl des jeweiligen Terminationsmixes (dNTP/-ddNTP) enthielten. Der A-Mix enthielt 1 mM dATP, 1 mM dCTP, 0,4 mM dGTP, 1,6 mM dITP, 1 mM dTTP, 5 µM ddATP; der C-Mix enthielt 1 mM dATP, 1 mM dCTP, 0,4 mM dGTP, 1,6mM dITP, 1 mM dTTP, 5 µM ddCTP; der G-Mix enthielt 1 mM dATP, 1 mM dCTP, 0,4 mM dGTP, 1,6mM dITP, 1 mM dTTP, 2,75 µM ddGTP und der T-Mix enthielt 1 mM dATP, 1 mM dCTP, 0,4 mM dGTP, 1,6mM dITP, 1 mM dTTP, 5 µM ddTTP. Die so angesetzten Reaktionen wurden wie folgt gecycelt: 30 mal mit den Schriften 95°C, 30''. 55°C, 20''; 68°C, 30''. Die Reaktionsgemische wurden bei 95°C für 10 Minuten im Thermocycler gänzlich eingedampft. Anschließend wurde 1 µl Loading Dye (Bestellnummer: 79448; Fa. Amersham) zugesetzt. Jeweils 0,2 µl jeder Reaktion (A, C, G, T) wurden auf verschiedene poröse Kamm-Materialien (Biodyne C^{R}, Loprodyne^{R}, Biodyne B^{R}, Porafil Membranfilter M-N) durch Applikation mit einer Piezo-Pipette geladen und auf die Elektrophorese-Apparatur der Fa. Li-Cor gebracht und die Elektrophorese wie im Li-Cor-Protokoll gemäß den Angaben des Herstellers durchgeführt. Die Experimente wurden mit dem AmplitaqFS-Kit der Fa. Perkin-Elmer durchgeführt.

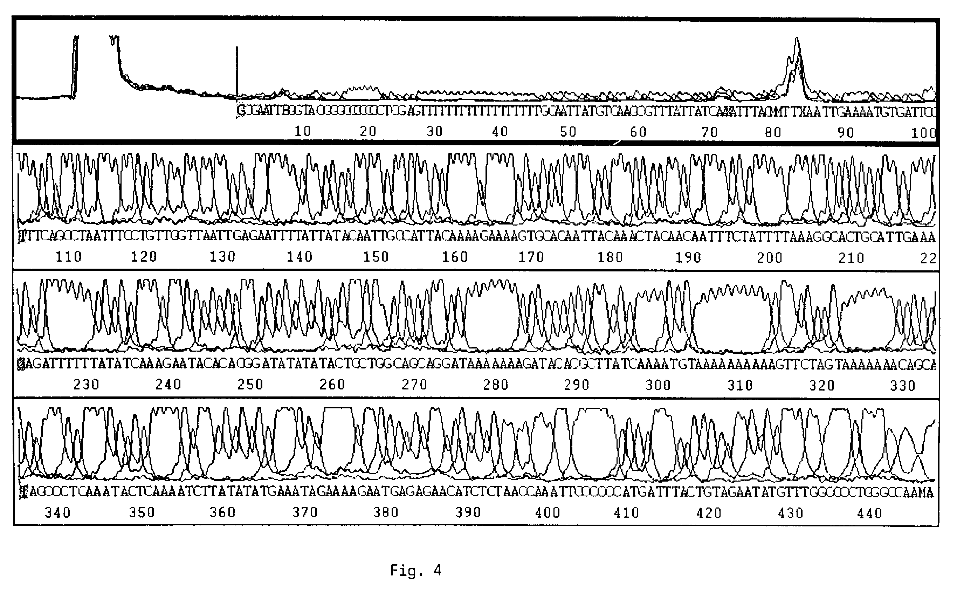

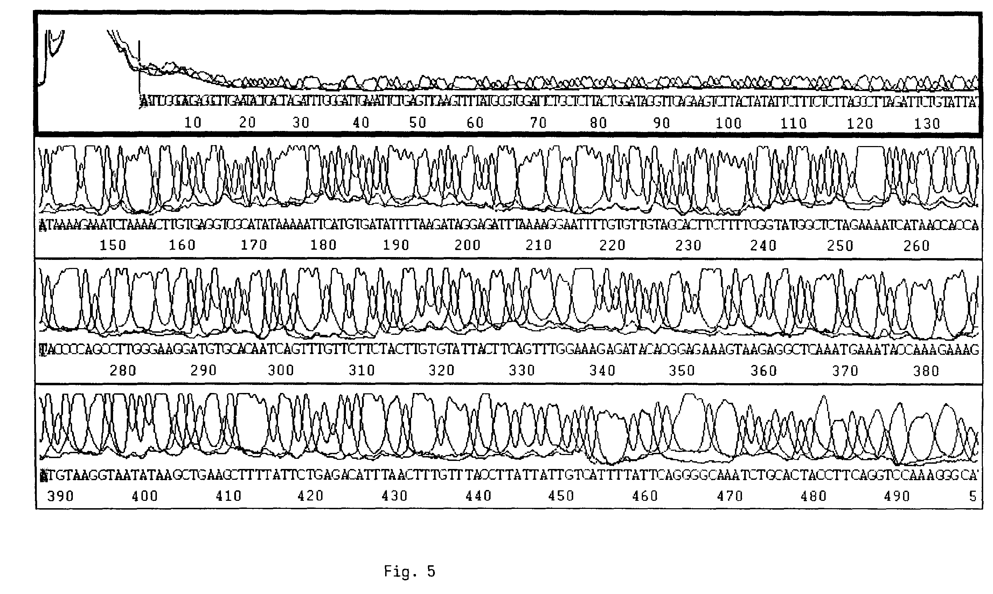

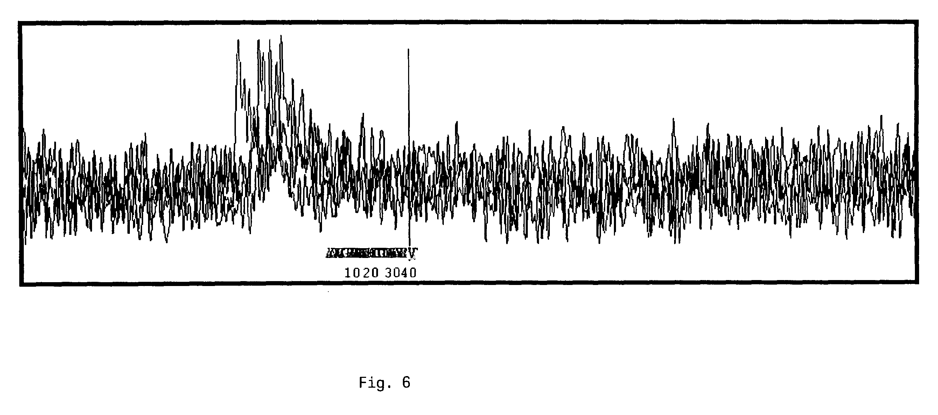

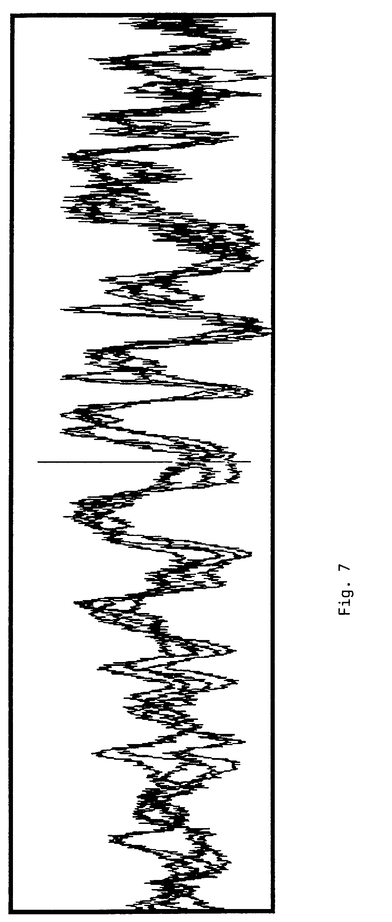

Die Ergebnisse der Sequenzierungen sind in den Fig. 4-7 gezeigt.

Zusammenfassend ist festzustellen, daß bei Einsatz einer Membran mit positiver Oberflächenladung schlechte Ergebnisse erzielt werden, während Membranen mit negativer Oberflächenladung beste Ergebnisse liefern.

## Patentansprüche

1. Verwendung von porösen hydrophilen Membranmaterialien, die an ihrer Oberfläche neutral oder negativ geladen sind, als Beladungsmaterialien bei der Gelelektrophorese von Nukleinsäuren oder Proteinen, die mit einem hydrophoben Farbstoff markiert sind.

2. Verwendung nach Anspruch 1, wobei die Membranen aus Nylon, Polyethersulfone, modifizierte Polyvinylidenfluoride, Cellulose-Acetat, Cellulose-Mischester oder regenerierte Cellulose sind.

3. Verwendung nach Anspruch 1 oder 2, wobei die Membranen die der Fig. 1 ausgewählt werden.

4. Verwendung nach einem der vorhergehenden Ansprüche, wobei der hydrophobe Farbstoff aus denen der Fig. 2 ausgewählt ist.

5. Verwendung nach einem der vorhergehenden Ansprüche, wobei die Membran eine mit Hydroxylgruppen modifizierte Nylon-Membran und der hydrophobe Farbstoff eine Emission im infraroten Bereich, insbesondere bei ca. 700-810 nm, zeigt.
